Europäisches Patentamt

European Patent Office

Office européen des brevets

(19)

(11) **EP 0 471 248 B1**

(12) **EUROPÄISCHE PATENTSCHRIFT**

(45) Veröffentlichungstag und Bekanntmachung des Hinweises auf die Patenterteilung:
**19.02.1997 Patentblatt 1997/08**

(51) Int Cl.6: **C08F 8/00**, C07C 17/275, C08F 10/10

(21) Anmeldenummer: **91113001.1**

(22) Anmeldetag: **02.08.1991**

(54) **Verfahren zur Herstellung von Kohlenwasserstoffen und Polymeren mit allylischen Chloridendgruppen**

Process for the production of hydrocarbons and polymers terminated by allylic chloride groups

Procédé pour la préparation d'hydrocarbures et de polymères ayant comme groupes terminaux des chlorures allyliques

(84) Benannte Vertragsstaaten:
**BE DE ES FR GB IT NL**

(30) Priorität: **16.08.1990 DE 4025961**
           **29.09.1990 DE 4030914**

(43) Veröffentlichungstag der Anmeldung:
**19.02.1992 Patentblatt 1992/08**

(73) Patentinhaber: **BASF Aktiengesellschaft 67063 Ludwigshafen (DE)**

(72) Erfinder:
• **Knoll, Konrad, Dr.**
  **W-6800 Mannheim 1 (DE)**
• **Bronstert, Klaus, Dr.**
  **W-6719 Carlsberg (DE)**
• **Bender, Dietmar, Dr.**
  **W-6707 Schifferstadt (DE)**

(56) Entgegenhaltungen:
EP-A- 0 265 053          EP-A- 0 380 052
WO-A-89/11494            US-A- 4 910 261

**Beschreibung**

Es ist bekannt, daß man durch kationische Polymerisation von Isobuten Oligomere oder Polymere mit endständiger Doppelbindung oder endständigem Chlor herstellen kann. Insbesondere weisen die Endgruppen die Struktur A

$$-CH_2-C(CH_3)_2-CH_2-C(CH_3)_2Cl \qquad (A)$$

auf. Solche Oligomeren oder Polymeren können z.B. unter Einsatz der sog. Inifer-Technik nach der US-PS 4,276,394 mit geeigneten mono- oder oligo-funktionellen Initiatoren hergestellt werden. Dabei erhält man unmittelbar Verbindungen, die als Endgruppen zwar an tertiären Kohlenstoff gebundenes Chlor enthalten, das aber für Folgereaktionen wenig geeignet ist.

Bevorzugte Ausgangsprodukte für Folgereaktionen sind dagegen solche, die durch Anlagerung von HCl an Oligomere oder Polymere entstehen, die ihrerseits aus Isobuten oder olefinischen $C_4$-Schnitten durch kationische Polymerisation hergestellt werden. Diese Ausgangsverbindungen sind z.B. mittels Katalysatoren wie $BF_3$ zugänglich, wie in der DE-A-2 904 314 beschrieben. Außerdem ist bekannt, daß man Isobuten auch kationisch oligomerisieren kann, wobei Olefine der Struktur B oder C

$$-CH_3-C(CH_3)_2-CH_2-C(=CH_2)-CH_3 \qquad (B)$$

oder

$$-CH_2-C(CH_3)_2-CH=C(CH_3)_2 \qquad (C)$$

entstehen. Diese kann man nach einem nicht vorveröffentlichten Vorschlag mit HCl in geeignete chlorhaltige Produkte umwandeln. Bei diesen Verfahren bzw. Reaktionen entstehen nämlich zunächst Verbindungen mit endständigem, an tertiäre C-Atome gebundenem Chlor, die als solche - wie erwähnt - für Folgereaktionen wenig geeignet sind. Insbesondere gelingt es nicht, den Chlor-Substituenten z.B. in primäres Hydroxyl, Amin, $-CH=CH_2$ oder Carboxyl umzuwandeln, da sowohl in alkalischem wie im sauren Medium sehr leicht HCl abgespalten wird und eine wenig reaktionsfähige Doppelbindung entsteht, die insbesondere im sauren Medium ins Innere der Kette hin wandert.

Die US-A 4 910 261 betrifft Blockcopolymere mit Polyisobuten-Mittelblöcken und Dien-Endblöcken jedoch ohne Chloratome und ohne Aminogruppen. In der EP-A 265 053 werden zwar chlorhaltige Polyolefine beschrieben, jedoch keine Polymeren mit dem erfindungswesentlichen Allylchlorid-Strukturelement $-CH_2-CH=CH-CH_2-Cl$. Die EP-A 380 052 betrifft Alkylhalogenide, jedoch wiederum ohne das Strukturelement $-CH_2-CH=CH-CH_2-Cl$. In der WO-A 89/11494 wird die Umsetzung von chlorierten Polyolefinen, die zusätzlich Carboxylgruppen enthalten, mit Hydroxylaminen beschrieben, jedoch kommt auch hier in den chlorierten Polyolefinen kein Strukturelement $-CH_2-CH=CH-CH_2-Cl$ vor.

Es bestand daher die Aufgabe, ein Verfahren anzugeben, das es ermöglicht, das tertiär gebundene Chlor so umzusetzen, daß dabei Endgruppen mit einer hohen Reaktivität entstehen, die mit einer Verbindung aus der Gruppe Ammoniak, primäres oder sekundäres Amin oder Polyamin, Aminoalkohol, Aminoether, Hydrazin oder bis zu dreifach substituiertem Hydrazin umgesetzt werden können.

Es wurde gefunden, daß diese Aufgabe in jeder Weise befriedigend gelöst wird, wenn man die tertiäres Chlor enthaltende Ausgangsprodukte in einem halogenhaltigen Kohlenwasserstoff mit Butadien in Gegenwart eines Friedel-Crafts-Katalysators wie $AlCl_3$, $ZnCl_2$, $SnCl_4$, $TiCl_4$ u.s.w., vorzugsweise jedoch Bortrichlorid umsetzt.

Unmittelbarer Erfindungsgegenstand ist ein Verfahren zur Herstellung von Kohlenwasserstoffen und Kohlenwasserstoffpolymeren der Struktur I

$$R-C(CH_3)_2-CH_2-C(CH_3)_2-CH_2-CH=CH-CH_2-Cl \qquad (I)$$

in der R einen Kohlenwasserstoffrest bezeichnet, gegebenenfalls im Gemisch mit entsprechenden Umsetzungsprodukten mit 2 oder mehr Mol Butadien pro Endgruppe, indem man erfindungsgemäß entsprechende, an tertiären Kohlenstoff gebundenes Chlor enthaltende Ausgangsprodukte der Struktur II

$$R-C(CH_3)_2-CH_2-C(CH_3)_2-Cl \qquad (II)$$

in Gegenwart von 1 bis 10 Äquivalenten eines Friedel-Crafts-Katalysators pro Endgruppe in einem gegenüber dem Katalysator indifferenten Lösungsmittel mit 1 bis 10 Mol Butadien umsetzt.

Dabei entsteht aus einer Struktur mit sehr labilem Chlor wie $R-CH_2C(CH_3)_2-Cl$ eine deutlich schlechter dissoziierende allylische Struktur $R-CH_2C(CH_3)_2-CH_2-CH=CH-CH_2-Cl$. Bei stöchiometrischer Butadienzugabe wird zwar die Addition von genau einer Butadieneinheit besonders begünstigt, jedoch bleiben statistisch einige Endgruppen unverändert. Bei geeigneter Reaktionsführung wird man daher 2 - 3 Äquivalente Butadien zur quantitativen Umsetzung aller Endgruppen mit tertiärem Chlorid verwenden, wobei ein Teil der Endgruppen auch 2 oder mehr Mol Butadien aufnimmt, die jedoch die gleichen guten Eigenschaften haben und die erfindungsgemäße Struktur des Kettenendes enthalten. Die neue Endgruppe ist in besonderem Maße für Substitutionsreaktionen unter Halogenaustausch prädestiniert. Mit dem allylisch gebundenen Chlor ist die Möglichkeit für Folgereaktionen für die Einführung einer großen Zahl von anderen Funktionalitäten eröffnet.

Vorteilhaft verwendet man als Ausgangsprodukte Oligomere oder Polymere des Isobutens, die durch kationische Polymerisation hergestellt wurden und an einem oder allen Kettenenden an tertiären Kohlenstoff gebundenes Chlor enthalten.

Eine besonders attraktive Variante des vorliegenden Verfahrens ist es, die Synthese bzw. Polymerisation der benötigten Cl-haltigen Ausgangsverbindungen mit dem gleichen Katalysator, also z.B. $BCl_3$ auszulösen, und dann die Umwandlung in die erfindungsgemäßen Verbindungen (I) im gleichen Reaktor vorzunehmen. Die Polymerisation mit $BCl_3$ ist z.B. in der US-PS 4,276,394 beschrieben.

Geeignete Lösungsmittel für Reaktionen unter Friedel-Crafts-Bedingungen sind z.B. Schwefelkohlenstoff, Nitrobenzol und im Falle von $ZnCl_2$ Ether wie Diethylether. Bei Temperaturen unter 50°C kommen chlorierte Kohlenwasserstoffe wie Dichlorethan, Trichlorethylen und Methylenchlorid in Frage.

Ein für die Katalyse mit $BCl_3$ besonders geeignetes Lösungsmittel ist Methylenchlorid. Das System $BCl_3$/$CH_2Cl_2$ erlaubt die quantitative Synthese der benötigten Cl-haltigen Ausgangsverbindungen (II) ebenso wie deren vollständige Umsetzung zu Produkten der Struktur (I). Gemische von chlorierten Lösungsmitteln sind ebenfalls verwendbar, auch können in gewissen Grenzen Zusätze an Kohlenwasserstoffen toleriert werden. Ein zu hoher Kohlenwasserstoffanteil beeinträchtigt die Selektivität der Reaktion.

Die Umsetzung verläuft im System $BCl_3$/$CH_2Cl_2$ i. a. im Bereich von -60 bis 20°C mit befriedigender Geschwindigkeit; bevorzugt ist eine Temperatur von 0°C oder tiefer.

1 bis 10 Äquivalente Friedel-Crafts-Katalysator werden pro Endgruppe eingesetzt, vorteilhaft sind 3 - 10 Äquivalente, hingegen bringen höhere Mengen keine Vorteile mehr; da Lösungsmittel und Katalysator schließlich zurückgewonnen werden können, spielt dies für die Wirtschaftlichkeit jedoch keine Rolle.

Dabei ist es ein wichtiger verfahrenstechnischer Vorteil, daß die Endgruppe von (I) relativ stabil gegen HCl-Eliminierung in Gegenwart von $BCl_3$ ist, im Gegensatz zu tertiären Chlorid-Endgruppen, die schon oberhalb -20°C HCl abzuspalten beginnen. Dies erlaubt eine effiziente Rückgewinnung des teuren $BCl_3$ und des Methylenchlorids, indem man einfach unter vermindertem Druck destilliert. Das Kondensat steht nach einer eventuellen Abtrennung von Spuren an HCl und nicht umgesetztem Butadien für einen neuen Einsatz zur Verfügung. Zur Erniedrigung der Viskosität des Polymerrückstands kann ein hochsiedendes, unter den Bedingungen inertes Lösungsmittel wie Isooktan oder Isododekan vor der Destillation beigemischt werden.

Gegenstand der vorliegenden Erfindung sind weiterhin Homopolyisobutylen mit Endgruppen der Struktur

$$-CH_2-CH=CH-CH_2-Cl$$

sowie polyisobutylen mit Endgruppen der Struktur

$$-CH_2-CH=CH-CH_2-R'$$

wobei R' einen Rest bedeutet, wie er durch Umsetzung einer Verbindung I in einem Lösungsmittel mittlerer Polarität mit überschüssigem Ammoniak oder einer Verbindung mit primären oder sekundären Aminogruppen aus der Gruppe primäre oder sekundäre Amine, Polyamine, Aminoalkohole, Aminoether, Hydrazin, alkylierte oder arylierte Hydrazine, wobei der Substitutionsgrad des Hydrazins bis zu drei betragen kann, Hydrazide und Hydrazone in Gegenwart einer Base erhalten wird.

Zur Charakterisierung der bei der Ausführung der nachstehenden Beispiele 1 - 3 erhaltenen Reaktionsprodukte wurden Chlorbestimmung, Gelpermeationschromatographie und ${}^1$H-NMR-Spektroskopie herangezogen. Die GPC-Messungen wurden auf einem Gerät der Fa. Waters mit einer Ultrastyragel-Säulenkombination derselben Firma durchgeführt. Die Detektion erfolgte mit einem Refraktometer, das an ein Computer-Auswertesystems der Fa. Polymer Standard Service gekoppelt war. Geeicht wurde mit Polyisobuten-Standards, Eluationsmittel war Tetrahydrofuran. Gemessen wurde $\overline{M}_n$ und $\overline{M}_w$.

Die ${}^1$H-NMR-Messungen wurden an einem 200 MHz-Gerät der Fa. Bruker bzw. an einem 60 MHz-Gerät der Fa. Jeol durchgeführt.

Bestimmung der $-CH_2-C(CH_3)_2-Cl$-Endgruppen

Die ${}^1$H-NMR-Spektroskopie erlaubt es, zwischen den Protonen der Endgruppe und denen der Hauptkette zu unterscheiden (Polymer Bulletin 3, 339 (1980); Polymer Bulletin 21,5 (1989)). Aus dem Intensitätsverhältnis der Resonanzen terminaler Methyl- und Methylengruppen bei $\delta$ = 1,67 bzw. 1,96 ppm sowie nicht-terminaler Methyl- und Methylengruppen bei $\delta$ = 1,1 bzw. 1,4 ppm wurde ebenfalls die mittlere Molmasse $\overline{M}_w$ berechnet.

Bestimmung der $-CH_2-CH=CH-CH_2-Cl$-Endgruppen

Die olefinischen Protonen der Endgruppe geben sich durch zwei charakteristische Multipletts des $ABX_2$-Spinsystems bei 5,6 und 5,8 ppm zu erkennen. Die Cl-tragende $CH_2$-Gruppe erscheint als Dublett mit Banden einer schwachen virtuellen Kopplung bei 4,0 ppm, während die allylische $CH_2$-Gruppe auf der Polyisobuten-Seite ein Dublett bei 2,0 ppm ergibt. Im Fall der Telechele kann aus dem Intensitätsverhältnis dieser Resonanzen zum Signal der olefinischen Initiatorsequenz bei 5,30 ppm auf den Grad der Umsetzung geschlossen werden. Ein weiteres wichtiges Indiz für eine vollständige Umsetzung ist das Verschwinden der Signale bei 1,67 bzw. 1,96 ppm, die von -CH$_2$-C(CH$_3$)$_2$-Cl-Gruppen herrühren.

Allgemeine Versuchsbedingungen

Alle Umsetzungen wurden unter Stickstoff, der mit einer Lösung von Butyllithium in 1,1-Diphenylethen/Mineralöl nachgereinigt war, in getrockneten Glasapparaturen durchgeführt. Methylenchlorid wurde unmittelbar vor der Benutzung von Triethylaluminium abdestilliert, das Isobuten durch überleiten über Molekularsieb nachgetrocknet.

Beispiel 1

1-Chlor-5,5,7,7-tetramethyl-2-okten und 1-Chlor-9,9,11,11-tetramethyl-2,6-dodekadien

In einem 500 ml Kolben werden 50 g 2-Chlor-2,4,4-trimethylpentan, 70 ml Dichlormethan und 50 ml Bortrichlorid bei -20°C vorgelegt, 10 Minuten gerührt und 19,5 g Butadien in 30 ml Dichlormethan innerhalb von 45 Minuten zugetropft; man rührt 3 h bei -20°C nach. Bortrichlorid und Dichlormethan werden bei 10 mbar abgezogen und der Rückstand mit 20 ml Methanol versetzt, das wieder abgezogen wird. Man schüttelt zwischen Pentan und Wasser/Natriumhydrogencarbonat aus und destilliert die Pentanphase: bei 22°C/l mbar gehen 9,24 g 2-Chlor-2-trimethylpentan über, bei 60°C/0,7 mbar 22,9 g (33,6 %) 1-Chlor-5,5,7,7-tetramethyl-2-okten, bei 115°C/0,7 mbar 7,65 g (8,9 %) 1-Chlor-9,9,11,11-tetramethyl-2,6-dodekadien. Es verbleibt 19,5 g Sumpf, der aus Mehrfachinsertionsprodukten des Butadiens besteht. Die zurückgewonnene Mischung aus Bortrichlorid und Dichlormethan kann mit gleichem Ergebnis wieder verwendet werden.

Beispiel 2

-CH$_2$-CH=CH-CH$_2$-Cl-terminiertes Polyisobuten-Makromer

In einem 4 l Rührreaktor mit Trockeneiskühler werden 2300 ml Dichlormethan, 130 ml Bortrichlorid und 77,4 g 2-Chlor-2-trimethylpentan für 5 Minuten bei -32°C miteinander vermischt und innerhalb 35 Minuten 800 ml Isobuten zugetropft und 60 Minuten nachpolymerisiert. Anschließend werden 108 ml Butadien zugegeben und 4 Stunden bei -28°C gerührt. Man fügt 300 ml Isooktan zu und zieht die flüchtigen Bestandteile bei -20°C i.v. ab. Es werden 100 ml Methanol zugetropft, die Mischung zwischen Pentan/Wasser ausgeschüttelt und die Pentanphase mit wäßriger Natriumhydrogencarbonat-Lösung neutral gestellt; man engt ein und trocknet bei 50°C im Ölpumpenvakuum. Cl-Analyse: 4 %; GPC: $\overline{M}_n$ = 1251 g/mol, $\overline{M}_w$ = 2276 g/mol, $\overline{M}_w/\overline{M}_n$ = 1,82.

Beispiel 3

-CH$_2$-CH=CH-CH$_2$-Cl-terminiertes Polyisobuten-Telechel

Die Umsetzung erfolgt wie in Beispiel 2 beschrieben. Es werden 1700 ml Dichlormethan, 55 ml Bortrichlorid, 35,3 g E-2,5-Dichlor-2,5-dimethyl-3-hexen, 600 ml Isobuten und 81 ml Butadien eingesetzt. Aufgearbeitet wird mit 50 ml Methanol und 250 ml Isooktan. Cl-Analyse: 4 %; GPC: $\overline{M}_n$ = 1933 g/mol, $\overline{M}_w$ = 2424 g/mol, $\overline{M}_w/\overline{M}_n$ = 1,25.

Die nach dem oben beschriebenen Verfahren hergestellten Kohlenwasserstoffe und Kohlenwasserstoffpolymere der Formel I sind als solche für Anwendungen als Kraftstoffadditive, Schmieröladditive, Klebstoffkomponenten und, mit telechel gebundenen Endgruppen, als Präpolymere für wasserdampfundurchlässige, chemisch inerte und lichtbeständige Polyharnstoffkautschuke ungeeignet. Sie werden indes zu interessanten Verbindungen, wenn sie statt mit der allylischen Chloridfunktion mit der entsprechenden allylischen Aminofunktion ausgerüstet sind.

Dies wird vorteilhaft erreicht, wenn man die Chloridendgruppen enthaltenden Kohlenwasserstoffe oder Kohlenwasserstoffpolymere in einem Lösungsmittel mittlerer Polarität mit überschüssigem Ammoniak oder einer Verbindung mit primären oder sekundären Aminogruppen in Gegenwart einer Base, z. B. eines Alkalioder Erdalkalioxids, -hydroxids oder -carbonats umsetzt. Das Amin substituiert die Chloridgruppe durch eine Aminogruppe, wobei HCl freigesetzt wird. Der Zusatz einer Base bewirkt die Freisetzung des Amins unter Bildung des entsprechenden Chlorids.

Als Verbindungen mit Aminfunktion kommen primäre oder sekundäre Amine, Polyamine, Aminoalkohole, Aminoether, Hydrazin, alkylierte oder arylierte Hydrazine, wobei der Substitutionsgrad des Hydrazins bis zu drei betragen kann, Hydrazide und Hydrazone in Betracht. Enthält die Aminokomponente mehr als eine NH-Bindung, ist es von Vorteil, mit einem Überschuß an Amin zu arbeiten, um Mehrfachalkylierung zu vermeiden. Günstig ist ein 5-15-facher Überschuß an Amin; Ammoniak sollte in mindestens 100-fachem Überschuß eingesetzt werden.

Bevorzugte Basen zur Bindung von HCl sind Magnesium- und Calciumoxid, Calciumhydroxid sowie Natrium- und Kaliumcarbonat.

Das Lösungsmittel wird nach Polarität und Menge so gewählt, daß das Amin und der Kohlenwasserstoff bzw. das Kohlenwasserstoffpolymere eine homogene Phase bilden, weil sonst beim Einsatz von Aminen mit mehr als einer N-H-Bindung Mehrfachalkylierung eintritt. Ist dies erwünscht, kann auch ohne Lösungsmittel gearbeitet werden. Besonders geeignete Lösungsmittel sind Ether wie Tetrahydrofuran, 1,4-Dioxan, Dimethoxyethan, Aromaten wie Benzol, Toluol oder Xylole, längerkettige Alkohole, prinzipiell auch chlorierte Kohlenwasserstoffe oder Mischungen aus den genannten Solventien. In Grenzen ist der Zusatz von aliphatischen Kohlenwasserstoffen tolerierbar.

Die Umsetzung wird vorteilhaft in einem Temperaturbereich zwischen 60 und 100°C durchgeführt; die Reaktionszeit beträgt dann 2 - 6 Stunden.

Bei Bedarf können schon während der Umsetzung Antioxidantien wie 2,6-Ditertiärbutylphenol u.ä. zugesetzt werden, um Braunfärbung und Verharzung zurückzudrängen.

Zur Aufarbeitung wird mit einem Kohlenwasserstoff wie Heptan, Isooktan oder Isododekan verdünnt, vom Niederschlag abgetrennt, was nicht zwingend notwendig ist, und mehrmals gegen eine nicht mischbare polare Phase extrahiert. Als polare Phase kommt Methanol, 25 %iges wäßriges Isopropanol, 5 %ige methanolische oder wäßrige Natronlauge in Frage. Der letzte Extraktionsgang sollte ohne Zusatz einer Base vorgenommen werden.

Die so hergestellten erfindungsgemäße Aminogruppen enthaltende Kohlenwasserstoffe bzw. Polymeren enthalten eine allylische Doppelbindung in der Nachbarschaft zur Aminogruppe. Diese kann bei Bedarf auf bekannte Weise hydriert werden. Geeignete Katalysatoren sind z.B. Raney-Nickel, Palladiumschwarz oder Platinschwarz; das Verfahren ist z.B. bei Houben-Weyl, Methoden der Organischen Chemie, Band IV/1c, G. Thieme Verlag, Stuttgart, 1980, Seite 18/19 beschrieben.

Zu den nachfolgenden Beispielen 4 - 14 ist folgendes vorauszuschicken:

Die eingesetzten Polymeren waren mit 0,1 % 2,6-Ditertiärbutylphenol stabilisiert. Die Amine wurden vor Verwendung frisch destilliert.

Zur Charakterisierung der Reaktionsprodukte wurden CHN-Analyse und $^1$H-NMR-Spektroskopie herangezogen. Die NMR-Messungen wurden an einem 60 MHz-Gerät der Fa. Jeol durchgeführt.

Zur Bestimmung des "$CH_2$-CH=CH-$CH_2$-Amin"-Restes muß man wissen, daß die olefinischen Protonen der Endgruppe sich als Multiplett bei 6,4 ppm zu erkennen geben. Die zum Aminstickstoff benachbarte allylische $CH_2$-Gruppe erscheint als Dublett bei 2,9 ppm, während die Resonanzen der allylischen $CH_2$-Gruppe auf der Polymerseite als Dublett bei 1,85 ppm auftreten. Direkt an den Stickstoff gebundene Protonen wurden gegebenenfalls durch H-D-Austausch mit Methanol-d[4] identifiziert.

Beispiel 4

1-Di-n-butylamino-5,5,7,7-tetramethyl-2-okten

10 g (49,5 mmol) 1-Chlor-5,5,7,7-tetramethyl-2-okten, 64 g (495 mmol) Di-n-butylamin und 10 g (248 mmol) Magnesiumoxid werden in 30 ml THF 4,5 h am Rückfluß gekocht. Man nimmt in 200 ml Hexan auf, extrahiert 2 mal gegen 5 %ige methanolische NaOH und einem gegen Wasser und trocknet bei 30°C/l mbar. Ausbeute: quantitativ

Beispiel 5

1-(N-(5,5,7,7-Tetramethyl-2-okten-1-yl)-2-aminoethyl) piperazin

Analog Beispiel 4 werden 10 g (49,5 mmol) 1-Chlor-5,5,7,7-tetramethyl-2-okten, 64 g (495 mmol) 1-(2-Aminoethyl)piperazin 10 g (248 mmol) Magnesiumoxid und 30 ml THF 4 h am Rückfluß gekocht. Man verdünnt mit 300 ml Diethylether und extrahiert je einmal gegen 5 %ige wäßrige NaOH und Wasser, engt ein und trocknet bei 40°C/1 mbar. Ausbeute: quantitativ

Beispiel 6

Umsetzung von chlorallylterminiertem Polyisobuten-Makromer mit 1-(2-Aminoethyl)piperazin

50 g 1-Chlor-4-Polyisobutenyl-2-buten der Molmasse 1000 (50 mmol), 64,6 g (500 mmol) 1-(2-Aminoethyl)piperazin und 10 g (250 mmol) Magnesiumoxid werden mit 25 ml THF 4 h am Rückfluß gehalten. Das THF wird im Vakuum abgezogen, der Rückstand in 100 ml Hexan aufgenommen und 3 x mit 40 ml absolutem Methanol extrahiert und die vereinigten Methanolextrakte einmal mit Hexan ausgeschüttelt. Die Lösung wird bei 40°C/0,2 mbar 3 h getrocknet. Umsatz: praktisch quantitativ.

Beispiel 7

Umsetzung von Makromer mit Hexamethylendiamin

Die umsetzung wird analog Beispiel 6 mit 58.1 g (0,5 mol) Hexamethylendiamin vorgenommen. Umsatz: praktisch quantitativ

Beispiel 8

umsetzung von Makromer mit Triethylentetramin

Die Umsetzung wird analog Beispiel 6 mit 73,1 g (0,5 mol) Triethylentetramin durchgeführt. Umsatz: praktisch quantitativ

Beispiel 9

Umsetzung von Makromer mit N-Methylethanolamin

Die Umsetzung wird analog Beispiel 6 mit 37,6 g (0,5 mol) N-Methylethanolamin durchgeführt. Umsatz: praktisch quantitativ.

Beispiel 10

Umsetzung wird analog Beispiel 6 mit 52,6 g (0,5 mol) Diethanolamin durchgeführt. Umsatz: praktisch quantitativ.

**Beispiel 11**

Umsetzung von Makromer mit Hydrazinhydrat

Die Umsetzung erfolgt analog Beispiel 6 mit 50 g (1 mol) Hydrazinhydrat. Umsatz: praktisch quantitativ.

**Beispiel 12**

Umsetzung von Makromer mit Phenylhydrazin

Die Umsetzung erfolgt analog Beispiel 6 mit 54 g (0,5 mol) Phenylhydrazin. Umsatz: praktisch quantitativ.

**Beispiel 13**

Umsetzung von Makromer mit $NH_3$

Ein 200 ml-Autoklav wird mit 10 g (10 mmol) Polymer, 10 g (187 mmol) Ammoniumchlorid und 50 ml Ethylenglykoldiethylether beschickt. Man kühlt mit Trockeneis und kondensiert 50 ml (2 mol) flüssigen Ammoniak ein. Der Autoklav wird verschlossen und 8 h auf 100°C erhitzt. Nach Abkühlen und Entspannen wird der Ether im Hochvakuum abgezogen, der Rückstand in 50 ml Pentan aufgenommen, über Kieselgur abgesaugt und das Filtrat im Hochvakuum getrocknet. Umsatz: praktisch quantitativ.

**Beispiel 14**

Umsetzung von chlorallylisch terminiertem Polyisobutenyl-Telechel mit 1-(2-Aminoethyl)piperazin

50 g (25 mmol) eines chlorallylisch terminierten Polyisobutentelechels der Molmasse Mn = 2000 werden analog Beispiel 6 mit 64,6 g (500 mmol) 1-(2-Aminoethyl)piperazin umgesetzt. Umsatz: praktisch quantitativ.

**Patentansprüche**

**Patentansprüche für folgende Vertragsstaaten : BE, DE, FR, GB, IT, NL**

1. Verfahren zur Herstellung von Kohlenwasserstoffen und Kohlenwasserstoffpolymeren der Struktur I

$$R-C(CH_3)_2-CH_2-C(CH_3)_2-CH_2-CH=$$

$$CH-CH_2-Cl \hspace{2cm} (I)$$

in der R einen Kohlenwasserstoffrest bezeichnet, gegebenenfalls im Gemisch mit entsprechenden Umsetzungsprodukten mit 2 oder mehr Mol Butadien pro Endgruppe, dadurch gekennzeichnet, daß man entsprechende, an tertiären Kohlenstoff gebundenes Chlor enthaltende Ausgangsprodukte der Struktur II

$$R-C(CH_3)_2-CH_2-C(CH_3)_2-Cl \hspace{1.5cm} (II)$$

in Gegenwart von 1-10 Äquivalenten eines Friedel-Crafts-Katalysators pro Endgruppe in einem gegenüber dem Katalysator indifferenten Lösungsmittel mit 1 bis 10 Mcl Butadien umsetzt.

2. Verfahren nach Anspruch 1, dadurch gekennzeichnet, daß man als Ausgangsprodukte Oligomere oder Polymere des Isobutens verwendet, die durch kationische Polymerisation hergestellt wurden und an einem oder allen Kettenenden an tertiären Kohlenstoff gebundenes Chlor enthalten.

3. Verfahren nach Anspruch 1, dadurch gekennzeichnet, daß man als Lösungsmittel Dichlormethan und je Mol Chlor enthaltender Endgruppe 1 bis 10 Mol $BCl_3$ als Friedel-Crafts-Katalysator einsetzt und bei einer Temperatur unter 0°C arbeitet.

4. Verfahren nach Anspruch 3, dadurch gekennzeichnet, daß der Katalysator $BCl_3$ und/oder das Lösungsmittel Dichlormethan nach der Umsetzung abdestilliert und zurückgeführt werden.

5. Homopolyisobutylen mit Endgruppen der Struktur

$$-CH_2-CH=CH-CH_2Cl.$$

6. Weitere Ausbildung des Verfahrens nach Anspruch 1, dadurch gekennzeichnet, daß man die Kohlenwasserstoff(polymeren) der Struktur I in einem Lösungsmittel mittlerer Polarität mit überschüssigem Ammoniak oder einer Verbindung mit primären oder sekundären Aminogruppen aus der Gruppe primäre oder sekundäre Amine, Polyamine, Aminoalkohole, Aminoether, Hydrazin, alkylierte oder arylierte Hydrazine, wobei der Substitutionsgrad des Hydrazins bis zu drei betragen kann, Hydrazide und Hydrazone in Gegenwart einer Base umsetzt.

7. Polyisobutylen mit Endgruppen der Struktur

$$CH_2-CH=CH-CH_2-R'$$

wobei R' einen Rest bedeutet, wie er durch Umsetzung einer Verbindung I gemäß Anspruch 1 in einem Lösungsmittel mittlerer Polarität mit überschüssigem Ammoniak oder einer Verbindung mit primären oder sekundären Aminogruppen aus der Gruppe primäre oder sekundäre Amine, Polyamine, Aminoalkohole, Aminoether, Hydrazin, alkylierte oder arylierte Hydrazine, wobei der Substitutionsgrad des Hydrazins bis zu drei betragen kann, Hydrazide und Hydrazone in Gegenwart einer Base erhalten wird.

**Patentansprüche für folgenden Vertragsstaat : ES**

1. Verfahren zur Herstellung von Kohlenwasserstoffen und Kohlenwasserstoffpolymeren der Struktur I

$$R-C(CH_3)_2-CH_2-C(CH_3)_2-CH_2-CH=$$

$$CH-CH_2-Cl \qquad (I)$$

in der R einen Kohlenwasserstoffrest bezeichnet, gegebenenfalls im Gemisch mit entsprechenden Umsetzungsprodukten mit 2 oder mehr Mol Butadien pro Endgruppe, dadurch gekennzeichnet, daß man entsprechende, an tertiären Kohlenstoff gebundenes Chlor enthaltende Ausgangsprodukte der Struktur II

$$R-C(CH_3)_2-CH_2-C(CH_3)_2-Cl \qquad (II)$$

in Gegenwart von 1-10 Äquivalenten eines Friedel-Crafts-Katalysators pro Endgruppe in einem gegenüber dem Katalysator indifferenten Lösungsmittel mit 1 bis 10 Mol Butadien umsetzt.

2. Verfahren nach Anspruch 1, dadurch gekennzeichnet, daß man als Ausgangsprodukte Oligomere oder Polymere des Isobutens verwendet, die durch kationische Polymerisation hergestellt wurden und an einem oder allen Kettenenden an tertiären Kohlenstoff gebundenes Chlor enthalten.

3. Verfahren nach Anspruch 1, dadurch gekennzeichnet, daß man als Lösungsmittel Dichlormethan und je Mol Chlor enthaltender Endgruppe 1 bis 10 Mol $BCl_3$ als Friedel-Crafts-Katalysator einsetzt und bei einer Temperatur unter 0°C arbeitet.

4. Verfahren nach Anspruch 3, dadurch gekennzeichnet, daß der Katalysator $BCl_3$ und/oder das Lösungsmittel Dichlormethan nach der Umsetzung abdestilliert und zurückgeführt werden.

5. Weitere Ausbildung des Verfahrens nach Anspruch 1, dadurch gekennzeichnet, daß man die Kohlenwasserstoff(polymeren) der Struktur I in einem Lösungsmittel mittlerer Polarität mit überschüssigem Ammoniak einer Verbindung mit primären oder sekundären Aminogruppen aus der Gruppe primäre oder sekundäre Amine, Polyamine, Aminoalkohole, Aminoether, Hydrazin, alkylierte oder arylierte Hydrazine, wobei der Substitutionsgrad des Hydrazins bis zu drei betragen kann, Hydrazide und Hydrozone in Gegenwart einer Base umsetzt.

**Claims**

**Claims for the following Contracting States : BE, DE, FR, GB, IT, NL**

1. A process for preparing hydrocarbons and polymers thereof, of the structure I

$$R-C(CH_3)_2-CH_2-C(CH_3)_2-CH_2-CH=$$

$$CH-CH_2-Cl \qquad (I)$$

where R is a hydrocarbon radical, if desired as a mixture with corresponding reaction products having 2 or more moles of butadiene per end group, which comprises reacting a corresponding starting material which contains chlorine bonded to a tertiary carbon, of the structure II

$$R-C(CH_3)_2-CH_2-C(CH_3)_2-Cl \qquad (II)$$

with from 1 to 10 moles of butadiene in the presence of from 1-10 equivalents of a Friedel-Crafts catalyst per end group in a solvent which is inert to the catalyst.

2. A process as claimed in claim 1, wherein the starting materials are isobutene oligomers or polymers prepared by cationic polymerization and containing chlorine bonded to tertiary carbon at one or all chain ends.

3. A process as claimed in claim 1, wherein the solvent is dichloromethane and from 1 to 10 moles of $BCl_3$ are employed as Friedel-Crafts catalyst per mole of chlorine-containing end group at below 0°C.

4. A process as claimed in claim 3, wherein the catalyst $BCl_3$ and/or the solvent dichloromethane are removed by distillation after the reaction and recycled.

5. A homopolyisobutylene with end groups of the structure

-CH$_2$-CH=CH-CH$_2$-Cl.

**6.** A further development of the process as claimed in claim 1, which comprises reacting the hydrocarbon (polymers) of the structure I in a solvent of average polarity with excess ammonia or a compound having primary or secondary amino groups from the group consisting of primary or secondary amines, polyamines, amino alcohols, amino ethers, hydrazine, alkylated or arylated hydrazines, it being possible for the hydrazine to have a degree of substitution of up to three, hydrazides and hydrazones, in the presence of a base.

**7.** A polyisobutylene with end groups of the structure

CH$_2$-CH=CH-CH$_2$-R'

where R' is a radical obtained by reacting a compound I as claimed in claim 1 in a solvent of average polarity with excess ammonia or a compound having primary or secondary amino groups from the group consisting of primary or secondary amines, polyamines, amino alcohols, amino ethers, hydrazine, alkylated or arylated hydrazines, it being possible for the hydrazine to have a degree of substitution of up to three, hydrazides and hydrazones, in the presence of a base.

**Claims for the following Contracting State : ES**

**1.** A process for preparing hydrocarbons and polymers thereof, of the structure I

R-C(CH$_3$)$_2$-CH$_2$-C(CH$_3$)$_2$-CH$_2$-CH=

CH-CH$_2$-Cl          (I)

where R is a hydrocarbon radical, if desired as a mixture with corresponding reaction products having 2 or more moles of butadiene per end group, which comprises reacting a corresponding starting material which contains chlorine bonded to a tertiary carbon, of the structure II

R-C(CH$_3$)$_2$-CH$_2$-C(CH$_3$)$_2$-Cl          (II)

with from 1 to 10 moles of butadiene in the presence of from 1-10 equivalents of a Friedel-Crafts catalyst per end group in a solvent which is inert to the catalyst.

**2.** A process as claimed in claim 1, wherein the starting materials are isobutene oligomers or polymers prepared by cationic polymerization and containing chlorine bonded to tertiary carbon at one or all chain ends.

**3.** A process as claimed in claim 1, wherein the solvent is dichloromethane and from 1 to 10 moles of BCl$_3$ are employed as Friedel-Crafts catalyst per mole of chlorine-containing end group at below 0°C.

**4.** A process as claimed in claim 3, wherein the catalyst BCl$_3$ and/or the solvent dichloromethane are removed by distillation after the reaction and recycled.

**5.** A further development of the process as claimed in claim 1, which comprises reacting the hydrocarbon (polymers) of the structure I in a solvent of average polarity with excess ammonia or a compound having primary or secondary amino groups from the group consisting of primary or secondary amines, polyamines, amino alcohols, amino ethers, hydrazine, alkylated or arylated hydrazines, it being possible for the hydrazine to have a degree of substitution of up to three, hydrazides and hydrazones, in the presence of a base.

**Revendications**

**Revendications pour les Etats contractants suivants : BE, DE, FR, GB, IT, NL**

**1.** Procédé de préparation d'hydrocarbures et de polymères d'hydrocarbures de structure I

R-C(CH$_3$)$_2$-CH$_2$-C(CH$_3$)$_2$-CH$_2$-CH=

CH-CH$_2$-Cl          (I)

dans laquelle R représente un reste hydrocarboné, éventuellement en mélange avec des produits de réaction correspondants comportant 2 moles de butadiène ou plus par groupement terminal, caractérisé en ce que l'on fait réagir des produits de départ correspondants, contenant un atome de chlore fixé sur un atome de carbone tertiaire, de structure II

R-C(CH$_3$)$_2$-CH$_2$-C(CH$_3$)$_2$-Cl          (II)

en présence de 1 à 10 équivalents d'un catalyseur de Friedel-Crafts par groupement terminal, dans un solvant inerte à l'égard du catalyseur, avec 1 à 10 moles de butadiène.

**2.** Procédé selon la revendication 1, caractérisé en ce que l'on utilise, comme produits de départ, des oligomères ou des polymères de l'isobutène qui ont été préparés par polymérisation cationique et contiennent, à une ou à toutes les extrémités de la chaîne, des atomes de chlore fixés sur des atomes de carbone tertiaires.

**3.** Procédé selon la revendication 1, caractérisé en ce que l'on utilise, comme solvant, du dichlorométhane et, comme catalyseur de Friedel-Crafts, 1 à 10 moles de $BCl_3$ par mole de groupement terminal contenant du chlore, et on procède à une température inférieure à 0°C.

**4.** Procédé selon la revendication 3, caractérisé en ce que le catalyseur $BCl_3$ et/ou le solvant dichlorométhane sont chassés par distillation après la réaction et réutilisés.

**5.** Homopolyisobutylène comportant des groupements terminaux de structure

$$-CH_2-CH=CH-CH_2Cl.$$

**6.** Développement du procédé selon la revendication 1, caractérisé en ce que l'on fait réagir l'hydrocarbure de structure I ou son polymère, dans un solvant de polarité moyenne, avec de l'ammoniac en excès ou un composé comportant des groupements amino primaires ou secondaires du groupe comprenant les amines primaires ou secondaires, les polyamines, les aminoalcools, les aminoéthers, l'hydrazine, les hydrazines alkylées ou arylées (le degré de substitution de l'hydrazine pouvant s'élever jusqu'à trois), les hydrazides et les hydrazones, en présence d'une base.

**7.** Polyisobutylène comportant des groupements terminaux de structure

$$-CH_2-CH=CH-CH_2-R'$$

dans laquelle R' représente un reste tel qu'obtenu par réaction d'un composé I selon la revendication 1, dans un solvant de polarité moyenne, avec de l'ammoniac en excès ou un composé comportant des groupements amino primaires ou secondaires du groupe comprenant les amines primaires ou secondaires, les polyamines, les aminoalcools, les aminoéthers, l'hydrazine, les hydrazines alkylées ou arylées (le degré de substitution de l'hydrazine pouvant s'élever jusqu'à trois), les hydrazides et les hydrazones, en présence d'une base.

**Revendications pour l'Etat contractant suivant : ES**

**1.** Procédé de préparation d'hydrocarbures et de polymères d'hydrocarbures de structure I

$$R-C(CH_3)_2-CH_2-C(CH_3)_2-CH_2-CH=$$

$$CH-CH_2-Cl \qquad (I)$$

dans laquelle R représente un reste hydrocarboné, éventuellement en mélange avec des produits de réaction correspondants comportant 2 moles de butadiène ou plus par groupement terminal, caractérisé en ce que l'on fait réagir des produits de départ correspondants, contenant un atome de chlore fixé sur un atome de carbone tertiaire, de structure II

$$R-C(CH_3)_2-CH_2-C(CH_3)_2-Cl \qquad (II)$$

en présence de 1 à 10 équivalents d'un catalyseur de Friedel-Crafts par groupement terminal, dans un solvant inerte à l'égard du catalyseur, avec 1 à 10 moles de butadiène.

**2.** Procédé selon la revendication 1, caractérisé en ce que l'on utilise, comme produits de départ, des oligomères ou des polymères de l'isobutène qui ont été préparés par polymérisation cationique et contiennent, à une ou à toutes les extrémités de la chaîne, des atomes de chlore fixés sur des atomes de carbone tertiaires.

**3.** Procédé selon la revendication 1, caractérisé en ce que l'on utilise, comme solvant, du dichlorométhane et, comme catalyseur de Friedel-Crafts, 1 à 10 moles de $BCl_3$ par mole de groupement terminal contenant du chlore, et on procède à une température inférieure à 0°C.

**4.** Procédé selon la revendication 3, caractérisé en ce que le catalyseur $BCl_3$ et/ou le solvant dichlorométhane sont chassés par distillation après la réaction et réutilisés.

**5.** Développement du procédé selon la revendication 1, caractérisé en ce que l'on fait réagir l'hydrocarbure de structure I ou son polymère, dans un solvant de polarité moyenne, avec de l'ammoniac en excès ou un composé comportant des groupements amino primaires ou secondaires du groupe comprenant les amines primaires ou secondaires, les polyamines, les aminoalcools, les aminoéthers, l'hydrazine, les hydrazines alkylées ou arylées (le degré de substitution de l'hydrazine pouvant s'élever jusqu'à trois), les hydrazides et les hydrazones, en présence d'une base.